(19) Europäisches Patentamt
European Patent Office
Office européen des brevets

(11) EP 1 273 583 A1

(12) EUROPEAN PATENT APPLICATION

(43) Date of publication:
**08.01.2003 Bulletin 2003/02**

(51) Int Cl.[7]: **C07D 403/06**

(21) Application number: **01116077.7**

(22) Date of filing: **03.07.2001**

(84) Designated Contracting States:
**AT BE CH CY DE DK ES FI FR GB GR IE IT LI LU MC NL PT SE TR**
Designated Extension States:
**AL LT LV MK RO SI**

(71) Applicant: **ICROM S.p.A.**
**20123 Milano (IT)**

(72) Inventor: **Leone, Mario**
**20067 Paullo (MI) (IT)**

(74) Representative: **Mancini, Vincenzo, Dr. et al**
**Ing. A. Giambrocono & C. s.r.l.,**
**Via Rosolino Pilo 19/B**
**20129 Milano (IT)**

(54) **Process for reducing 2-(11H-dibenz(b,e)azepin-6-ylmethyl)-1H-isoindole-1,3(2H)-dione**

(57) A process for reducing 2-(11H-dibenz|b,e|azepin-6-ylmethyl)-1H-isoindole-1,3(2H)-dione to 2-(6,11-dihydro-5H-dibenz|b,e|azepin-6yl-methyl)-1H-isoindole-1,3(2H)-dione is disclosed, which comprises carrying out the reduction, in an organic solvent, in the presence of a metallic catalyst and of formic acid and/or at least one pharmaceutically acceptable salt thereof; the process is safe and allows to obtain a nearly quantitative conversion without the need of a high pressure.

EP 1 273 583 A1

## Description

The invention relates to a process for reducing 2-(11H-dibenz|b,e| azepin-6-ylmethyl)-1H-isoindole-1,3(2H)-dione. Particularly, the invention concerns the reduction of 2-(11H-dibenz|b,e| azepin-6-ylmethyl)-1H-isoindole-1,3(2H)-dione to 2-(6,11-dihydro-5H-dibenz|b,e| azepin-6yl-methyl)-1H-isoindole-1,3(2H)-dione, which is a useful intermediate in the synthesis of important antihistaminic and antidepressant drugs.

The reduction of 2-(11H-dibenz|b,e| azepin-6-ylmethyl)-1H-isoindole-1,3(2H)-dione (hereinafter compound A, see scheme I) is disclosed in EP-A-496,306; the resulting product, i.e. 2-(6,11-dihydro-5H-dibenz|b,e| azepin-6yl-methyl)-1H-isoindole-1,3(2H)-dione (hereinafter compound B, see scheme I) is, as above noted, a useful intermediate for the synthesis of important drugs such as epinastine and mianserine. The disclosed reduction is a well-known hydrogenation, performed either in dimethylformamide and 0.5-5 mol/mol of formic acid or in dimethylacetamide and 0.5-5 mol/mol of acetic acid and at 1-10 bar of hydrogen pressure, at a temperature of 40-100°C, in the presence of palladium on charcoal, as illustrated by the following scheme I:

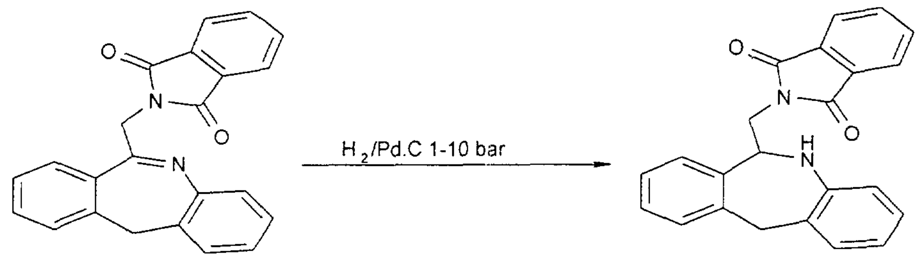


(A) (B)

It is well known, however that hydrogen is a highly dangerous gas (see, for instance, Kirk-Othmer, Encyclopedia of Chemical Technology, vol. 12, Wiley-Interscience NY 3rd ed., 1980) and, therefore, performing high pressure hydrogenation is not quite safe on an industrial scale and requires special equipments and technologies which, of course, increases dramatically the industrial cost of the bulk intermediates.

Hydrogen Transfer Catalysis (hereinafter HCT) is a well known procedure, safer than under pressure hydrogenation, and is an alternative to the use of hydrogen gas; this technology contemplates the same catalyst as the common hydrogenation yet replaces molecular hydrogen with a hydrogen source or donor.

A number of hydrogen donors are described in literature: cyclohexene [S.A. Khan, Synthesis, 751, (1978); A.E. Jackson, Synthesis, 685, (1976)], hydrazine [S.A. Khan, Synthesis, 751, (1978); B.M. Adger, Synthesis, 53, (1987)], sugars [G. Brieger, J. Chem. Soc. Chem. Comm., 757, (1976); G. Brieger, J. Org. Chem., 44, 1876, (1979)], isopropanol [R.A. W. Johnstone, Chem. Rev., 85, 129 (1985); V.S. Rao, Can. J. Chem., 61, 652, (1983)], formic acid and formates [S. Ram, Synth. Comm., 17, 415, (1987); T. Bieg, Synthesis, 76, (1985)]. No one of the above mentioned donors produces hydrogen gas and there is no need of pressure, so HCT is a chemical reaction that does neither require any special apparatus nor particular safety equipments.

The wide range of HCT procedure described in the prior art concerning either the reduction of the nitroaromatic moiety or the cleavage of the benzyl protecting group and, what is most, the observation that unsatured carbon-nitrogen bonds are not hydrogenated by HCT [M. Bertau, Chemistry Today, 17, 51, (1999)] taught the skilled in the art that this attractive alternative to catalytic hydrogenation could not be carried out.

It has now been found, surprisingly, that 2-(11H-dibenz|b,e| azepin-6-ylmethyl)-1H-isoindole-1,3(2H)-dione (compound A) is indeed reduced by HCT.

Accordingly, a process for reducing compound A, to compound B is disclosed, which comprises carrying out the reduction, in an organic solvent, in the presence of a metallic catalyst, wherein the metal is selected among the elements belonging to group VIII B of the periodic table, and of 5-20 moles, per mole of compound A, of formic acid and/or at least one pharmaceutically acceptable salt thereof.

Preferably, the metallic catalyst is a metal in its elemental state, particularly palladium on activated charcoal, most preferably in an amount of 10% calculated on the dry catalyst. It is however appropriate to use also an *in situ* reduceable salt of the above defined metal such as, for instance $PdCl_2$, as an alternative or in combination with the metal in its elemental state.

The reduction is carried out in an organic, protic or aprotic, solvent such as, for instance, at least one of the following: chlorinated solvents, such as dichloromethane, chlorobenzene, freon 11, 22, 23, chloroform and 2,2,2-trifluoroethanol, ethy] acetate, dimethyl sulfoxide, sulfolane, formic acid, acetic acid, dimethylformamide, dimethylacetamide; dimethylacetamide and/or dimethylformamide, in an amount of 50-200 g of compound A per liter, are preferred.

The process of the invention can be carried out at a temperature between 40° and 140°C, preferably between

60° and 120°C.

It is also preferred to carry out the process of the invention with formic acid in admixture with at least one pharmaceutically acceptable salt thereof. Examples of pharmaceutically acceptable salts of formic acid are the salts with metals of the I and the II group of the periodic table, such as sodium, potassium, calcium and magnesium or the ammonium salt; the salts with primary amines, such as methylamine, ethylamine, propylamine and ciclohexylamine; the salts with secondary amines such as dimethylamine, diethylamine and diisopropylamine; the salts with tertiary amines such as trimethylamine, triethylamine, ethyldiisopropylamine, dimethylaniline and dimethylaminopyridine; the salts with aromatic amines such as pyridine, 2-methylpyridine, 3-methylpyridine, 4-methylpyridine and 2,6-dimethylpyridine; ammonium formate is particularly preferred.

The process of the invention results to be safe, since there is no development of any dangerous gas, and allows to obtain compound B with a nearly quantitative conversion, avoiding, at the same time, side reactions which derive, for instance, from hydride reduction; further, there is neither the need of a high pressure, nor of special equipments and technologies.

The process of the invention is illustrated by the following scheme II:

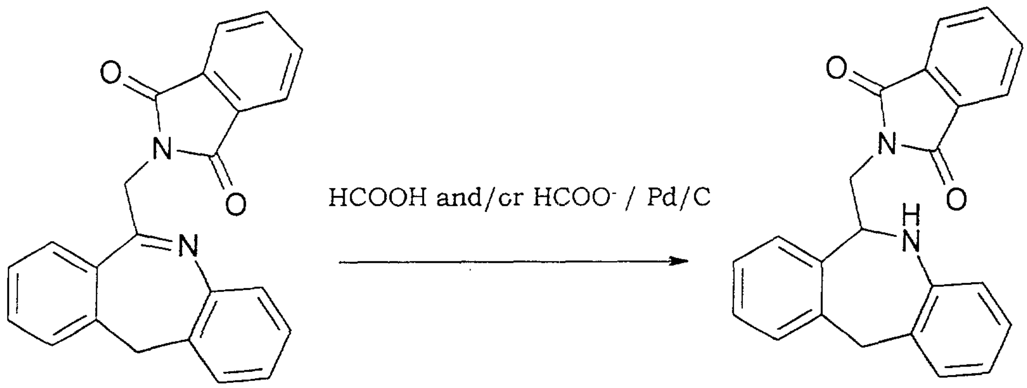


(A) (B)

Scheme II

Compound (A) is dissolved in an organic solvent, preferably selected among at least one of the above mentioned solvents; the choice of the solvent does not affect the course of the reaction and is only related to the solubility of the substrate. Also the amount of the solvent is evidently mainly related to the solubility, the reaction being possibly performed without specific attention to choosing a peculiar concentration, although an amount equal to or above 10 g, particularly 50-200 g, of compound A per liter is to be preferred.

Formic acid and/or a pharmaceutically salt thereof can be used to carry out the process of the invention; a mixture of formic acid with at least one of said salts is preferred.

The pH of the solution ranges preferably between 3 and 9, more preferably between 4 and 8. Lower pHs can slow the reaction whereas higher pHs can cause some degradation. In some cases, the production of a carbonate may occur, for instance ammonium carbonate when ammonium formate is used, therefore checking pH can assist adjusting pH within the above mentioned range.

The total amount of formic acid and/or of the pharmaceutically acceptable salt thereof ranges between 5 and 20 moles per mole of the compound A.

The catalyst, as above mentioned, is preferably metallic palladium on activated charcoal even if it is also suitable to use a palladium salt, such as $PdCl_2$ which is reduced *in situ* during the reaction, as an alternative or in combination with metallic palladium, in an amount of the metal which is 10% calculated on the dry catalyst.

The following examples illustrate the invention without limiting it.

EXAMPLE 1

The starting material was well known in the literature (CAS r.n. 74860-00-7), the reactions were monitored by reverse phase HPLC on a Lichrosorb® 5 μ, RP 18 column.

5.5 g of compound A, were dissolved in 100 ml of dimethylacetamide, 0.08 mol of formic acid and 0.08 mol of ammonia were added, the resulting mixture was stirred and 0.4 mmol of palladium on charcoal were added. The

reaction was kept at 80°C and stirred for 3 hours, after that further 0.08 mol of formic acid were added keeping the resulting mixture at the same temperature until the HPLC analysis showed less than 2% of the starting material. The catalyst was filtered off and washed with fresh dimethylacetamide, the mother liquor being concentrated and diluted with water. The precipitate was harvested under vacuum with a Buchner funnel and, after drying, 5 g of compound (B) were obtained (92% of the theoretical value).

EXAMPLE 2

The procedure of Example 1 was repeated replacing dimethylacetamide with dimethylformamide; also in this case, a yield of 92% of the theoretical value was obtained.

### Claims

1. A process for reducing 2-(11H-dibenz|b,e| azepin-6-ylmethyl)-1H-isoindole-1,3(2H)-dione, to 2-(6,11-dihydro-5H-dibenz|b,e| azepin-6yl-methyl)-1H-isoindole-1,3(2H)-dione, which comprises carrying out the reduction, in an organic solvent, in the presence of a metallic catalyst, wherein the metal is selected among the elements belonging to group VIII B of the periodic table, and of 5-20 moles, per mole of 2-(11H-dibenz|b,e| azepin-6-ylmethyl)-1H-isoindole-1,3(2H)-dione, of formic acid and/or at least one pharmaceutically acceptable salt thereof.

2. Process according to claim 1, wherein the catalyst is palladium on activated charcoal and/or an *in situ* reduceable salt thereof.

3. Process according to claim 1 or 2, wherein the catalyst is palladium on activated charcoal, in an amount of 10% calculated on the dry catalyst.

4. Process according to any of the previous claims, wherein the solvent is selected among at least one of the following: dichloromethane, chlorobenzene, freon 11, 22, 23, chloroform, 2,2,2-trifluoroethanol, ethyl acetate, dimethyl sulfoxide, sulfolane, formic acid, acetic acid, dimethylformamide, dimethylacetamide.

5. Process according to any of the previous claims, wherein the solvent is dimethylacetamide and/or dimethylformamide, in an amount of 50-200 g of compound A per liter.

6. Process according to any of the previous claims, wherein the pharmaceutically acceptable salt of formic acid is selected among the salts with the metals of the I and the II group of the periodic table or the ammonium salt; the salts with primary, secondary or tertiary amines; and the salts with aromatic amines.

7. Process according to any of the previous claims, wherein the pharmaceutically acceptable salt of formic acid is selected among the salts with sodium, potassium, calcium, magnesium, ammonium, methylamine, ethylamine, propylamine, ciclohexylamine, dimethylamine, diethylamine, diisopropylamine, trimethylamine, triethylamine, ethyldiisopropylamine, dimethylaniline, dimethylaminopyridine, pyridine, 2-methylpyridine, 3-methylpyridine, 4-methylpyridine and 2,6-dimethylpyridine.

8. Process according to any of the previous claims, wherein the pharmaceutically acceptable salt of formic acid is ammonium formate.

9. Process according to any of the previous claims, which is carried out at a temperature between 40° and 140°C.

10. Process according to any of the previous claims, which is carried out at a temperature between 60° and 120°C.

11. Process according to any of the previous claims, which is carried out at a pH ranging between 3 and 9.

12. Process according to any of the previous claims, which is carried out at a pH ranging between 4 and 8.

European Patent Office

# EUROPEAN SEARCH REPORT

Application Number
EP 01 11 6077

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| Y | I. KALDOR ET AL.: "Stereocontrolled Synthesis of cis-Dibenzoquinolizine Chlorofumarates: Curare-Like Agents of Ultrashort Duration" J. ORG. CHEM., vol. 66, no. 10, 2001, pages 3495-3501, XP002192469 * Scheme 1 * * page 3500 * | 1-12 | C07D403/06 |
| Y | G. MEUZELAAR ET AL.: "Chemistry of Opium Alkaloids, 45. Improvements in the Total Synthesis of Morphine" EUR. J. ORG. CHEM., vol. 9, 1999, pages 2315-2321, XP002192470 * Scheme 3* * page 2320 * | 1-12 | |
| Y | L. F. TIETZE ET AL.: "Synthesis of Simple Enantiopure Tetrahydro-.beta.-carbolines and Tetrahydroisoquinolines" EUR. J. ORG. CHEM., vol. 12, 2000, pages 2247-2252, XP002192471 * Scheme 4* | 1-12 | TECHNICAL FIELDS SEARCHED (Int.Cl.7) C07D |
| Y | E. VEDEJS ET AL.: "Substituted Isoquinolines by Noyori Transfer Hydrogenation: Enantioselective Synthesis of Chiral Diamines Containing an Aniline Subunit" J. ORG. CHEM., vol. 64, no. 18, 1999, pages 6724-6729, XP002192472 * Scheme 2 * | 1-12 | |

-/--

The present search report has been drawn up for all claims

| Place of search | Date of completion of the search | Examiner |
|---|---|---|
| MUNICH | 11 March 2002 | Herz, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

European Patent Office

# EUROPEAN SEARCH REPORT

Application Number
EP 01 11 6077

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| Y | A. A. BANERJEE, D. MUKESH: "Heterogeneous Catalytic Transfer Hydrogenation of 4-Nitrodiphenylamine to p-Phenylenediamines" J. CHEM. SOC. CHEM. COMMUN., vol. 18, 1988, pages 1275-1276, XP001057766 * Scheme 2 * --- | 1-12 | |
| Y | Y. WATANABE ET AL.: "Ruthenium Catalyzed Reduction of Nitroarenes and Azaaromatic Compounds Using Formic Acid" BULL. CHEM. SOC. JPN., vol. 57, no. 9, 1984, pages 2440-2444, XP001057848 * page 2442 * --- | 1-12 | |
| Y | R. BALTZLY, O. KAUDER: "The preparation of secondary amines through the Wallach reaction" J. ORG. CHEM., vol. 16, 1951, pages 173-177, XP001057811 * page 176 * --- | 1-12 | TECHNICAL FIELDS SEARCHED (Int.Cl.7) |
| Y | R. LUKES, O. CERVINKA: "Syntheses in the allo-lupinane series. III. A note on the preparation of 4-hydroxymethylquinolizidine" COLLECT. CZECH. CHEM. COMMUN., vol. 24, 1959, pages 1846-1850, XP001057833 * page 1848 * ----- | 1-12 | |

The present search report has been drawn up for all claims

| Place of search | Date of completion of the search | Examiner |
|---|---|---|
| MUNICH | 11 March 2002 | Herz, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)